# EUROPEAN PATENT APPLICATION

(11) **EP 4 509 495 A1**
(43) Date of publication of application: **19.02.2025**
(21) Application number: 23788356.6
(22) Date of filing: 12.04.2023
(51) Int. Cl.: C07D 213/61, C07D 213/80

(54) **NOVEL PYRIDINIUM COMPOUND**

(30) Priority: 13.04.2022 JP 2022066550
(71) Applicant: Kao Corporation, Tokyo 103-8210 (JP)
(72) Inventor: INAGAKI Kensuke, Wakayama-shi, Wakayama 640-8580 (JP); Takano Ninna, Nagoya-shi, Aichi 466-0828 (JP)
(74) Representative: Hoffmann Eitle
(86) International application number: PCT/JP2023/014845
(87) International publication number: WO 2023/199938

(57) **Abstract**

The present invention is a novel pyridinium compound represented by the following general formula (1), (2) or (3): 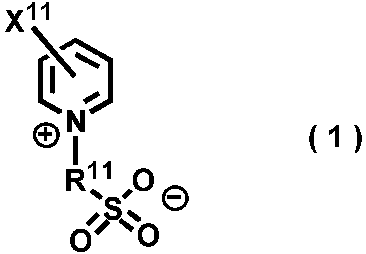 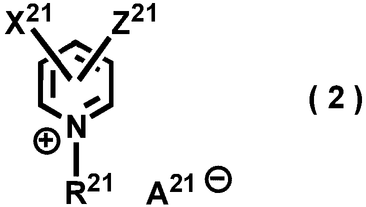 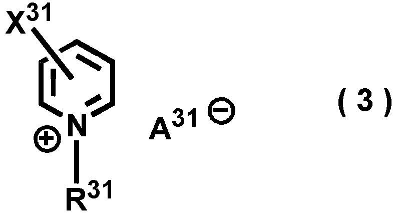 wherein X¹¹, X²¹ and X³¹ each represent a halogen atom, X¹¹ X²¹ and X³¹ each being bonded to a carbon atom at position 2, 4 or 6 of the pyridine ring,
R¹¹ is an alkylene group with 1 or more and 24 or less carbons which may contain a heteroatom,
Z²¹ is a group selected from -COO-R²², -CONH-R²³ and -CON(R²⁴)(R²⁵), Z²¹ being bonded to a carbon atom of the pyridine ring different from the carbon atom to which X²¹ is bonded,
R²¹ is an alkyl group with 3 or more and 24 or less carbons which may contain a heteroatom,
R²², R²³, R²⁴ and R²⁵ each represent an alkyl group with 3 or more carbons which may contain a heteroatom,
R³¹ is a branched alkyl group with 10 or more and 24 or less carbons which may contain a heteroatom, and
A²¹⁻ and A³¹⁻ each represent an anion.

## Description

### Field of the Invention

The present invention relates to a novel pyridinium compound.

### Background of the Invention

Pyridinium compounds are compounds containing cations derived from pyridine, and the compounds are chemically modified as necessary and used in various applications such as bleaching, agricultural chemicals, pharmaceuticals, sterilization, reagents for synthesis, catalysts and others.

JP-A 2008-120699 discloses a pharmaceutical containing a specific pyridinium derivative formed of a naphthalene ring bonded to a pyridinium ring having an aryl group or the like bonded at position 1 thereof.

JP-A S60-112761 discloses a specific novel pyridinium compound produced from a specific pyridinium compound.

On the other hand, while oxidizing agents such as hydrogen peroxide and others are used as so-called oxygen bleach, bleach activators may be used in combination with them to strengthen bleaching power. As bleach activators, such compounds as to react with hydrogen peroxide to produce organic peroxy acids (activated species) are known.

### Summary of the Invention

It is considered that, if a novel compound that can be used as such a bleach activator as to react with hydrogen peroxide to produce an organic peroxy acid (activated species) can be provided, a more desirable situation in terms of design flexibility and others can be realized.

The present invention provides a novel pyridinium compound.

The present invention relates to a novel pyridinium compound represented by the general formula (1), (2) or (3) below. Hereinafter, the novel pyridinium compounds represented by the general formula (1), (2) and (3) below are referred to as compounds (1), (2) and (3), respectively. 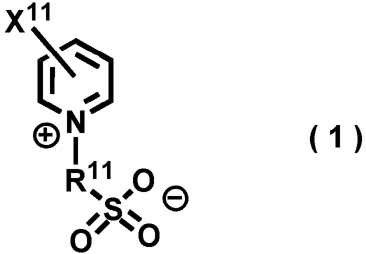 wherein X¹¹ is a halogen atom, X¹¹ being bonded to a carbon atom at position 2, 4 or 6 of the pyridine ring, and R¹¹ is an alkylene group with 1 or more and 24 or less carbons which may contain a heteroatom. 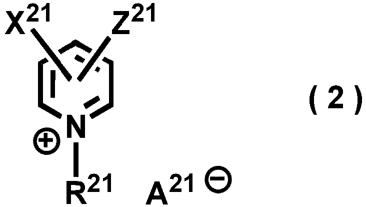 wherein X²¹ is a halogen atom, X²¹ being bonded to a carbon atom at position 2, 4 or 6 of the pyridine ring, Z²¹ is a group selected from -COO-R²², -CONH-R²³ and - CON(R²⁴)(R²⁵), Z²¹ being bonded to a carbon atom of the pyridine ring different from the carbon atom to which X²¹ is bonded, R²¹ is an alkyl group with 3 or more and 24 or less carbons which may contain a heteroatom, R²², R²³, R²⁴ and R²⁵ each represent an alkyl group with 3 or more carbons which may contain a heteroatom, and A²¹⁻ is an anion. 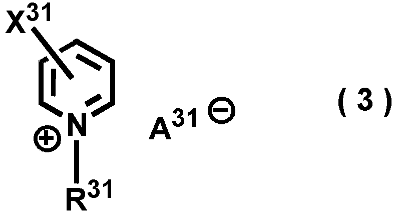 wherein X³¹ is a halogen atom, X³¹ being bonded to a carbon atom at position 2, 4 or 6 of the pyridine ring, R³¹ is a branched alkyl group with 10 or more and 24 or less carbons which may contain a heteroatom, and A³¹⁻ is an anion.

According to the present invention, a novel pyridinium compound is provided.

The pyridinium compound of the present invention can be expected to be utilized as an efficacy enhancing agent for oxidizing agents, for example, peroxy acid oxidizing agents such as hydrogen peroxide and others, or the like.

### Embodiments of the Invention

### <Compound (1)>

Compound (1) is a pyridinium compound represented by the above general formula (1).

In the general formula (1), X¹¹ is a halogen atom, and X¹¹ is bonded to a carbon atom at position 2, 4 or 6 of the pyridine ring. Examples of the halogen atom include a fluorine atom, a chlorine atom, a bromine atom and an iodine atom, and a chlorine atom is preferable. Note that the position numbers of the pyridine ring are as follows:

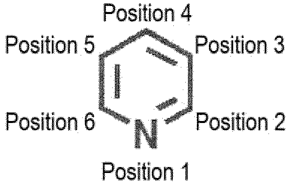

In the general formula (1), R¹¹ is an alkylene group with 1 or more and 24 or less carbons which may contain a heteroatom. R¹¹ has, for example, 2 or more and further 3 or more, and 20 or less, further 18 or less, further 16 or less, further 14 or less, further 12 or less, further 10 or less, further 8 or less, further 6 or less and further 4 or less carbons. Examples of the heteroatom include, for example, a nitrogen atom, an oxygen atom, a sulfur atom and a phosphorus atom. When R¹¹ is an alkylene group containing a heteroatom, the number of carbons in R¹¹ may be a total number of carbons in the carbon chain excluding the heteroatom. R¹¹ may be an alkylene group with 1 or more and 24 or less carbons not containing a heteroatom.

In the general formula (1), examples of -R¹¹-SO₃ include, for example, -CH₂-(CH₂)ₙ₁₁-Y¹-(CH₂)ₙ₁₂-SO₃. Here, Y¹ is a single bond or a heteroatom, n11 and n12 each represent an integer of 0 or more and 23 or less, and a total of n11 and n12 is 0 or more and 23 or less.

Compound (1) can be synthesized, for example, by a reaction of a pyridine halide with a sultone. As illustrated in the Examples described later, compound (1) can be synthesized, for example, by reacting 2-chloropyridine with 1,3-propanesultone.

The synthesis of compound (1) can be carried out in the absence of a solvent or in the presence of a reaction solvent. As the reaction solvent, for example, dichloromethane, dichloroethane, chloroform, diethyl ether, tetrahydrofuran, dioxane, diglyme, dimethoxyethane, cyclopentyl methyl ether, dibutyl ether, cyclohexane, acetone, acetonitrile, methyl ethyl ketone, ethyl acetate, dimethylformamide, benzene, toluene, xylene, trifluoromethylbenzene, nitrobenzene or the like can be used.

A reaction temperature during the synthesis of compound (1) is preferably 0°C or more and more preferably 25°C or more, and preferably 100°C or less and more preferably 60°C or less from the viewpoint of improving a yield of product.

After the reaction, processes such as extraction, purification, collection and others of compound (1) can be carried out as necessary, and for example, column chromatography, preparative TLC, recrystallization, wash with solvent and others can be used for those processes.

### <Compound (2)>

Compound (2) is a pyridinium compound represented by the above general formula (2).

In the general formula (2), X²¹ is a halogen atom, and X²¹ is bonded to a carbon atom at position 2, 4 or 6 of the pyridine ring. Examples of the halogen atom include a fluorine atom, a chlorine atom, a bromine atom and an iodine atom, and a chlorine atom is preferable.

In the general formula (2), Z²¹ is a group selected from -COO-R²², -CONH-R²³ and -CON(R²⁴)(R²⁵), and Z²¹ is bonded to a carbon atom of the pyridine ring different from the carbon atom to which X²¹ is bonded. R²², R²³, R²⁴ and R²⁵ each represent an alkyl group with 3 or more carbons which may contain a heteroatom. R²², R²³, R²⁴ and R²⁵ each have, for example, 4 or more, further 8 or more and further 12 or more, and 24 or less, further 20 or less and further 18 or less carbons. Examples of the heteroatom include, for example, a nitrogen atom, an oxygen atom, a sulfur atom and a phosphorus atom. When R²², R²³, R²⁴ and R²⁵ each represent an alkyl group containing a heteroatom, the numbers of carbons in R²², R²³, R²⁴ and R²⁵ may each be a total number of carbons in the carbon chain excluding the heteroatom. R²², R²³, R²⁴ and R²⁵ may each be an alkyl group with the above number of carbons not containing a heteroatom. Z²¹ is preferably a group selected from -COO-R²² and -CONH-R²³,

In the general formula (2), R²¹ is an alkyl group with 3 or more and 24 or less carbons which may contain a heteroatom. R²¹ has, for example, 4 or more, further 8 or more and further 12 or more, and 24 or less, further 20 or less and further 18 or less carbons. Examples of the heteroatom include, for example, a nitrogen atom, an oxygen atom, a sulfur atom and a phosphorus atom. When R²¹ is an alkyl group containing a heteroatom, the number of carbons in R²¹ may be a total number of carbons in the carbon chain excluding the heteroatom. R²¹ may be an alkyl group with 3 or more and 24 or less carbons not containing a heteroatom.

In the general formula (2), examples of -R²¹ include, for example, -CH₂-(CH₂)ₙ₂₁-Y²-(CH₂)ₙ₂₂-CH₃. Here, Y² is a single bond or a heteroatom, n21 and n22 each represent an integer of 0 or more and 22 or less, and a total of n21 and n22 is 1 or more and 22 or less.

In the general formula (2), A²¹⁻ is an anion. The anion of A²¹⁻ may be either an organic anion or an inorganic anion. Examples of the anion of A²¹⁻ include, for example, an anion which is a conjugate base of an acid with a pKa of 5 or less. Examples of the anion of A²¹⁻ include, for example, sulfonic acid ions such as a trifluoromethanesulfonate ion (⁻OTf), a para toluenesulfonate ion (⁻OTs), a methanesulfonate ion (⁻OMs) and others, alkyl sulfate ions such as a methyl sulfate ion (⁻OSO₃Me) and others, halide ions such as a chloride ion (Cl⁻), a bromide ion (Br⁻), an iodide ion (I⁻) and others, fluoroborate ions such as a tetrafluoroborate ion (BF₄⁻) and others, fluorophosphate ions such as a hexafluorophosphate ion (PF₆⁻) and others, imide ions such as a bis(trifluoromethanesulfonyl)imide ion ((CF₃SO₂)₂N⁻) and others, fluoroacetate ions such as a trifluoroacetate ion (CF₃COO⁻) and others, or the like. A²¹⁻ is preferably an anion selected from ⁻OTf, ⁻OSO₃Me,⁻OTs, ⁻OMs, Cl⁻ and Br⁻.

Of compound (2), a compound of the general formula (2) in which Z²¹ is -COO-R²² can be synthesized, for example, by reacting 2 chloronicotinic acid chloride with an alcohol such as 1-butanol, dodecanol, 2-decyl-1-tetradecanol or the like to obtain an ester compound and quaternizing the ester compound with a quaternizing agent such as butyl trifluoromethanesulfonate, dodecyl trifluoromethanesulfonate or the like, as illustrated in the Examples described later.

Further, of compound (2), a compound of the general formula (2) in which Z²¹ is -CONH-R²³ can be synthesized, for example, by reacting 2 chloronicotinic acid chloride with an alkyl amine such as butylamine or the like to obtain an amide compound and quaternizing the amide compound with a quaternizing agent such as butyl trifluoromethanesulfonate, dodecyl trifluoromethanesulfonate or the like, as illustrated in the Examples described later.

A reaction solvent can be used for the synthesis of compound (2). As the reaction solvent, for example, dichloromethane, dichloroethane, chloroform, diethyl ether, tetrahydrofuran, dioxane, diglyme, dimethoxyethane, cyclopentyl methyl ether, dibutyl ether, cyclohexane, acetone, acetonitrile, methyl ethyl ketone, ethyl acetate, dimethylformamide, benzene, toluene, xylene, trifluoromethylbenzene, nitrobenzene or the like can be used.

A reaction temperature during the synthesis of compound (2) is preferably 0°C or more and more preferably 25°C or more, and preferably 100°C or less and more preferably 60°C or less from the viewpoint of improving a yield of product.

After the reaction, processes such as extraction, purification, collection and others of compound (2) can be carried out as necessary, and for example, column chromatography, preparative TLC, recrystallization, wash with solvent and others can be used for those processes.

### <Compound (3)>

Compound (3) is a pyridinium compound represented by the above general formula (3).

In the general formula (3), X³¹ is a halogen atom, and X³¹ is bonded to a carbon atom at position 2, 4 or 6 of the pyridine ring. Examples of the halogen atom include a fluorine atom, a chlorine atom, a bromine atom and an iodine atom, and a chlorine atom is preferable.

In the general formula (3), R³¹ is a branched alkyl group with 10 or more and 24 or less carbons which may contain a heteroatom. R³¹ has, for example, 12 or more, further 16 or more and further 20 or more, and 28 or less, further 24 or less and further 22 or less carbons. Examples of the heteroatom include, for example, a nitrogen atom, an oxygen atom, a sulfur atom and a phosphorus atom. When R³¹ is an alkyl group containing a heteroatom, the number of carbons in R³¹ may be a total number of carbons in the carbon chain excluding the heteroatom. R³¹ may be a branched alkyl group with 10 or more and 24 or less carbons not containing a heteroatom. R³¹ is preferably a branched alkyl group having a methyl branch and further preferably a branched alkyl group having multiple methyl branches from the viewpoint of availability.

In the general formula (3), examples of -R³¹ include, for example, -CH₂-R³¹¹-Y³-R³¹²-CH₃. Here, Y³ is a single bond or a heteroatom, R³¹¹ and R³¹² each represent a single bond or an alkylene group, a total number of carbons in R³¹¹ and R³¹² is 8 or more and 22 or less, and at least one of R³¹¹ and R³¹² is a branched alkylene group.

In the general formula (3), A³¹⁻ is an anion. The anion of A³¹⁻ may be either an organic anion or an inorganic anion. Examples of the anion of A³¹⁻ include, for example, an anion which is a conjugate base of an acid with a pKa of 5 or less. Examples of the anion of A³¹⁻ include, for example, sulfonic acid ions such as a trifluoromethanesulfonate ion (⁻OTf), a para toluenesulfonate ion (⁻OTs), a methanesulfonate ion (⁻OMs) and others, alkyl sulfate ions such as a methyl sulfate ion (⁻OSO₃Me) and others, halide ions such as a chloride ion (Cl⁻), a bromide ion (Br⁻), an iodide ion (I⁻) and others, fluoroborate ions such as a tetrafluoroborate ion (BF₄⁻) and others, fluorophosphate ions such as a hexafluorophosphate ion (PF₆⁻) and others, imide ions such as a bis(trifluoromethanesulfonyl)imide ion ((CF₃SO₂)₂N⁻) and others, fluoroacetate ions such as a trifluoroacetate ion (CF₃COO⁻) and others, or the like. A³¹⁻ is preferably an anion selected from ⁻OTf, ⁻OSO₃Me,⁻OTs, ⁻OMs, Cl⁻ and Br⁻.

Compound (3) can be synthesized, for example, by reacting an alcohol such as 3,7-dimethyloctanol, 3,7,11,15-tetramethylhexadecanol or the like with trifluoromethanesulfonic anhydride to obtain a sulfonic ester and reacting the sulfonic ester with 2-chloropyridine, as illustrated in the Examples described later.

A reaction solvent can be used for the synthesis of compound (3). As the reaction solvent, for example, dichloromethane, dichloroethane, chloroform, diethyl ether, tetrahydrofuran, dioxane, diglyme, dimethoxyethane, cyclopentyl methyl ether, dibutyl ether, cyclohexane, acetone, acetonitrile, methyl ethyl ketone, ethyl acetate, dimethylformamide, benzene, toluene, xylene, trifluoromethylbenzene, nitrobenzene or the like can be used.

A reaction temperature during the synthesis of compound (3) is preferably 0°C or more and more preferably 25°C or more, and preferably 100°C or less and more preferably 60°C or less from the viewpoint of improving a yield of product.

After the reaction, processes such as extraction, purification, collection and others of compound (3) can be carried out as necessary, and for example, column chromatography, preparative TLC, recrystallization, wash with solvent and others can be used for those processes.

Compounds (1) to (3) of the present invention can be expected to be utilized directly or indirectly for applications such as, for example, bleaching, decolorization, reactants, washing, deodorization, antivirals, sterilization, spore killing and others in a wide variety of fields. For example, compounds (1) to (3) of the present invention can be expected to be utilized directly or indirectly for each of the following:
(I) applications such as bleaching of various types of pulp, deinking and bleaching of waste paper, and others in the pulp and paper industry;
(II) applications such as bleaching of natural fibers such as cotton, wool, silk or the like and synthetic fibers or the like, and others in the textile industry;
(III) applications such as raw materials for organic and inorganic peroxides, raw materials for organic compounds, raw materials for epoxy compounds, and others in the chemical engineering field;
(IV) applications such as etching and cleaning of semiconductors or printed circuit boards, and others in the electronic engineering field;
(V) applications such as deodorization, sterilization and decolorization treatments of sewage or industrial wastewater, soil improvement, and others in the pollution treatment field;
(VI) applications such as oxidation of metals in refining processes, and others in the mining field;
(VII) applications such as sterilization of initial product manufacturing equipment, sterilization of containers, bleaching of food, and others in the food field;
(VIII) applications such as intermediate raw materials for pharmaceuticals, and others in the pharmaceutical field;
(IX) applications such as surface treatment of metals, purification of plating solutions, and others in the metal finishing field; and
(X) applications such as bleaching of wood, bleaching of decorative laminates, and others in the wood field.

The present invention provides use of the novel pyridinium compound represented by the above general formula (1), the novel pyridinium compound represented by the above general formula (2) or the novel pyridinium compound represented by the above general formula (3) as an oxidative decomposition activator. The present invention provides use of the novel pyridinium compound represented by the above general formula (1), the novel pyridinium compound represented by the above general formula (2) or the novel pyridinium compound represented by the above general formula (3) in the production of an oxidative decomposition activator. The present invention provides use of the novel pyridinium compound represented by the above general formula (1), the novel pyridinium compound represented by the above general formula (2) or the novel pyridinium compound represented by the above general formula (3) in the production of an oxidizing agent. The present invention further provides an oxidative decomposition activator composed of the novel pyridinium compound represented by the above general formula (1), the novel pyridinium compound represented by the above general formula (2) or the novel pyridinium compound represented by the above general formula (3).

Further, the present invention provides use of the novel pyridinium compound represented by the above general formula (1), the novel pyridinium compound represented by the above general formula (2) or the novel pyridinium compound represented by the above general formula (3) as a bleach activator. The present invention provides use of the novel pyridinium compound represented by the above general formula (1), the novel pyridinium compound represented by the above general formula (2) or the novel pyridinium compound represented by the above general formula (3) in the production of a bleach activator. The present invention provides use of the novel pyridinium compound represented by the above general formula (1), the novel pyridinium compound represented by the above general formula (2) or the novel pyridinium compound represented by the above general formula (3) in the production of a bleaching agent. The present invention further provides a bleach activator composed of the novel pyridinium compound represented by the above general formula (1), the novel pyridinium compound represented by the above general formula (2) or the novel pyridinium compound represented by the above general formula (3).

The matters stated in compounds (1) to (3) of the present invention can be appropriately applied to these uses, oxidative decomposition activator and bleach activator.

In addition to the aforementioned embodiments, the present invention discloses the aspects below.
<1> A novel pyridinium compound represented by the following general formula (1), (2) or (3): 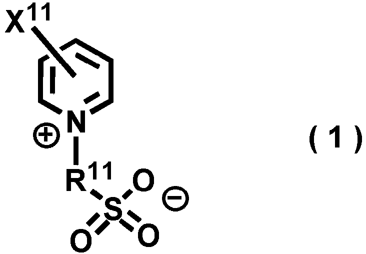
   wherein X¹¹ is a halogen atom, X¹¹ being bonded to a carbon atom at position 2, 4 or 6 of the pyridine ring, and R¹¹ is an alkylene group with 1 or more and 24 or less carbons which may contain a heteroatom; 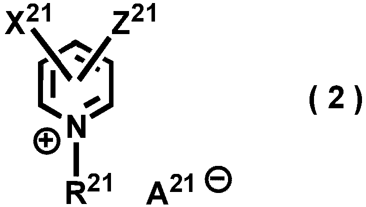 wherein X²¹ is a halogen atom, X²¹ being bonded to a carbon atom at position 2, 4 or 6 of the pyridine ring, Z²¹ is a group selected from -COO-R²², -CONH-R²³ and - CON(R²⁴)(R²⁵), Z²¹ being bonded to a carbon atom of the pyridine ring different from the carbon atom to which X²¹ is bonded, R²¹ is an alkyl group with 3 or more and 24 or less carbons which may contain a heteroatom, R²², R²³, R²⁴ and R²⁵ each represent an alkyl group with 3 or more carbons which may contain a heteroatom, and A²¹⁻ is an anion; or 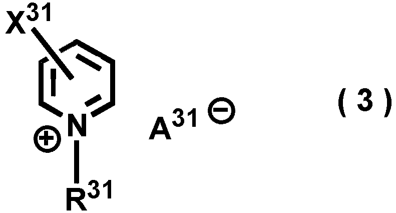
   wherein X³¹ is a halogen atom, X³¹ being bonded to a carbon atom at position 2, 4 or 6 of the pyridine ring, R³¹ is a branched alkyl group with 10 or more and 24 or less carbons which may contain a heteroatom, and A³¹⁻ is an anion.
<2> The compound according to <1>, wherein in the general formula (1), X¹¹ is a halogen atom selected from a fluorine atom, a chlorine atom, a bromine atom and an iodine atom, and further a chlorine atom.
<3> The compound according to <1> or <2>, wherein in the general formula (1), R¹¹ has 2 or more and further 3 or more, and 20 or less, further 18 or less, further 16 or less, further 14 or less, further 12 or less, further 10 or less, further 8 or less, further 6 or less and further 4 or less carbons.
<4> The compound according to any of <1> to <3>, wherein in the general formula (1), the heteroatom in R¹¹ is at least one selected from a nitrogen atom, an oxygen atom, a sulfur atom and a phosphorus atom.
<5> The compound according to any of <1> to <4>, wherein in the general formula (1), R¹¹ is an alkylene group with 1 or more and 24 or less carbons not containing a heteroatom.
<6> The compound according to any of <1> to <5>, wherein in the general formula (1), -R¹¹-SO₃ is -CH₂-(CH₂)ₙ₁₁-Y¹-(CH₂)ₙ₁₂-SO₃, Y¹ is a single bond or a heteroatom, n11 and n12 each represent an integer of 0 or more and 23 or less, and a total of n11 and n12 is 0 or more and 23 or less.
<7> The compound according to any of <1> to <6>, wherein in the general formula (1), X¹¹ is a chlorine atom, X¹¹ being bonded to a carbon atom at position 2 or 6 of the pyridine ring, and R¹¹ is an alkylene group with 3 carbons.
<8> The compound according to any of <1> to <7>, wherein in the general formula (2), X²¹ is a halogen atom selected from a fluorine atom, a chlorine atom, a bromine atom and an iodine atom, and further a chlorine atom.
<9> The compound according to any of <1> to <8>, wherein in the general formula (2), R²², R²³, R²⁴ and R²⁵ each have 4 or more, further 8 or more and further 12 or more, and 24 or less, further 20 or less and further 18 or less carbons.
<10> The compound according to any of <1> to <9>, wherein in the general formula (2), the heteroatoms in R²², R²³, R²⁴ and R²⁵ are each at least one selected from a nitrogen atom, an oxygen atom, a sulfur atom and a phosphorus atom.
<11> The compound according to any of <1> to <10>, wherein in the general formula (2), R²², R²³, R²⁴ and R²⁵ each represent an alkyl group with the above number of carbons not containing a heteroatom.
<12> The compound according to any of <1> to <11>, wherein in the general formula (2), Z²¹ is a group selected from -COO-R²² and -CONH-R²³.
<13> The compound according to any of <1> to <12>, wherein in the general formula (2), R²¹ has 4 or more, further 8 or more and further 12 or more, and 24 or less, further 20 or less and further 18 or less carbons.
<14> The compound according to any of <1> to <13>, wherein in the general formula (2), the heteroatom in R²¹ is at least one selected from a nitrogen atom, an oxygen atom, a sulfur atom and a phosphorus atom.
<15> The compound according to any of <1> to <13>, wherein in the general formula (2), R²¹ is an alkyl group with 3 or more and 24 or less carbons not containing a heteroatom.
<16> The compound according to any of <1> to <14>, wherein in the general formula (2), -R²¹ is -CH₂-(CH₂)ₙ₂₁-Y²-(CH₂)ₙ₂₂-CH₃, Y² is a single bond or a heteroatom, n21 and n22 each represent an integer of 0 or more and 22 or less, and a total of n21 and n22 is 1 or more and 22 or less.
<17> The compound according to any of <1> to <16>, wherein in the general formula (2), A²¹⁻ is an anion selected from an organic anion and an inorganic anion.
<18> The compound according to any of <1> to <17>, wherein in the general formula (2), A²¹⁻ is an anion which is a conjugate base of an acid with a pKa of 5 or less.
<19> The compound according to any of <1> to <18>, wherein in the general formula (2), A²¹⁻ is an anion selected from a sulfonic acid ion, an alkyl sulfate ion, a halide ion, a fluoroborate ion, a fluorophosphate ion, an imide ion and a fluoroacetate ion, and further an anion selected from a trifluoromethanesulfonate ion (⁻OTf), a para toluenesulfonate ion (⁻OTs), a methanesulfonate ion (⁻OMs), a methyl sulfate ion (⁻OSO₃Me), a chloride ion (Cl⁻), a bromide ion (Br⁻), an iodide ion (I⁻), a tetrafluoroborate ion (BF₄⁻), a hexafluorophosphate ion (PF₆⁻), a bis(trifluoromethanesulfonyl)imide ion ((CF₃SO₂)₂N⁻) and a trifluoroacetate ion (CF₃COO⁻) .
<20> The compound according to any of <1> to <19>, wherein in the general formula (2), A²¹⁻ is an anion selected from ⁻OTf, ⁻OSO₃Me, ⁻OTs, ⁻OMs, Cl⁻ and Br⁻.
<21> The compound according to any of <1> to <20>, wherein in the general formula (2), X²¹ is a chlorine atom, X²¹ being bonded to a carbon atom at position 2 of the pyridine ring, Z²¹ is a group selected from -COO-R²² and -CONH-R²³, Z²¹ being bonded to a carbon atom of the pyridine ring different from the carbon atom to which X²¹ is bonded, R²¹ is a group selected from a butyl group, a dodecyl group and a tetracosyl group, and A²¹ is ⁻OTf.
<22> The compound according to any of <1> to <21>, wherein in the general formula (2), X²¹ is a chlorine atom, X²¹ being bonded to a carbon atom at position 2 of the pyridine ring, Z²¹ is -COO-R²², Z²¹ being bonded to a carbon atom of the pyridine ring different from the carbon atom to which X²¹ is bonded, R²² is a group selected from a butyl group, a dodecyl group and a 2-decyltetradecyl group, R²¹ is a group selected from a butyl group, a dodecyl group and a tetracosyl group, and A²¹ is ⁻OTf.
<23> The compound according to any of <1> to <22>, wherein in the general formula (2), X²¹ is a chlorine atom, X²¹ being bonded to a carbon atom at position 2 of the pyridine ring, Z²¹ is -COO-R²², in which R²² is a butyl group, Z²¹ being bonded to a carbon atom at position 3 of the pyridine ring, R²¹ is a dodecyl group, and A²¹ is⁻OTf.
<24> The compound according to any of <1> to <22>, wherein in the general formula (2), X²¹ is a chlorine atom, X²¹ being bonded to a carbon atom at position 2 of the pyridine ring, Z²¹ is -COO-R²², in which R²² is a dodecyl group, Z²¹ being bonded to a carbon atom at position 3 of the pyridine ring, R²¹ is a dodecyl group, and A²¹ is ⁻OTf.
<25> The compound according to any of <1> to <22>, wherein in the general formula (2), X²¹ is a chlorine atom, X²¹ being bonded to a carbon atom at position 2 of the pyridine ring, Z²¹ is -COO-R²², in which R²² is a 2-decyltetradecyl group, Z²¹ being bonded to a carbon atom at position 3 of the pyridine ring, R²¹ is a dodecyl group, and A²¹ is ⁻OTf.
<26> The compound according to any of <1> to <22>, wherein in the general formula (2), X²¹ is a chlorine atom, X²¹ being bonded to a carbon atom at position 2 of the pyridine ring, Z²¹ is -COO-R²², in which R²² is a dodecyl group, Z²¹ being bonded to a carbon atom at position 3 of the pyridine ring, R²¹ is a butyl group, and A²¹ is ⁻OTf.
<27> The compound according to any of <1> to <22>, wherein in the general formula (2), X²¹ is a chlorine atom, X²¹ being bonded to a carbon atom at position 2 of the pyridine ring, Z²¹ is -COO-R²², in which R²² is a dodecyl group, Z²¹ being bonded to a carbon atom at position 3 of the pyridine ring, R²¹ is a tetracosyl group, and A²¹ is ⁻OTf.
<28> The compound according to any of <1> to <21>, wherein in the general formula (2), X²¹ is a chlorine atom, X²¹ being bonded to a carbon atom at position 2 of the pyridine ring, Z²¹ is -CONH-R²³, Z²¹ being bonded to a carbon atom of the pyridine ring different from the carbon atom to which X²¹ is bonded, R²³ is a butyl group, R²¹ is a group selected from a butyl group and a dodecyl group, and A²¹ is ⁻OTf.
<29> The compound according to any of <1> to <21>, wherein in the general formula (2), X²¹ is a chlorine atom, X²¹ being bonded to a carbon atom at position 2 of the pyridine ring, Z²¹ is -CONH-R²³, in which R²³ is a butyl group, Z²¹ being bonded to a carbon atom at position 3 of the pyridine ring, R²¹ is a butyl group, and A²¹ is ⁻OTf.
<30> The compound according to any of <1> to <21>, wherein in the general formula (2), X²¹ is a chlorine atom, X²¹ being bonded to a carbon atom at position 2 of the pyridine ring, Z²¹ is -CONH-R²³, in which R²³ is a butyl group, Z²¹ being bonded to a carbon atom at position 3 of the pyridine ring, R²¹ is a dodecyl group, and A²¹ is ⁻OTf.
<31> The compound according to any of <1> to <30>, wherein in the general formula (3), X³¹ is a halogen atom selected from a fluorine atom, a chlorine atom, a bromine atom and an iodine atom, and further a chlorine atom.
<32> The compound according to any of <1> to <31>, wherein in the general formula (3), R³¹ has 12 or more, further 16 or more and further 20 or more, and 28 or less, further 24 or less and further 22 or less carbons.
<33> The compound according to any of <1> to <32>, wherein in the general formula (3), the heteroatom in R³¹ is at least one selected from a nitrogen atom, an oxygen atom, a sulfur atom and a phosphorus atom.
<34> The compound according to any of <1> to <33>, wherein in the general formula (3), R³¹ is a branched alkyl group with 10 or more and 24 or less carbons not containing a heteroatom, further a branched alkyl group with 10 or more and 24 or less carbons having a methyl branch, and further a branched alkyl group with 10 or more and 24 or less carbons having multiple methyl branches.
<35> The compound according to any of <1> to <34>, wherein in the general formula (3), -R³¹ is -CH₂-R³¹¹-Y³-R³¹²-CH₃, Y³ is a single bond or a heteroatom, R³¹¹ and R³¹² each represent a single bond or an alkylene group, a total number of carbons in R³¹¹ and R³¹² is 8 or more and 22 or less, and at least one of R³¹¹ and R³¹² is a branched alkylene group.
<36> The compound according to any of <1> to <35>, wherein in the general formula (3), A³¹⁻ is an anion selected from an organic anion and an inorganic anion.
<37> The compound according to any of <1> to <36>, wherein in the general formula (3), A³¹⁻ is an anion which is a conjugate base of an acid with a pKa of 5 or less.
<38> The compound according to any of <1> to <37>, wherein in the general formula (3), A³¹⁻ is an anion selected from a sulfonic acid ion, an alkyl sulfate ion, a halide ion, a fluoroborate ion, a fluorophosphate ion, an imide ion and a fluoroacetate ion, further an anion selected from a trifluoromethanesulfonate ion (⁻OTf), a para toluenesulfonate ion (⁻OTs), a methanesulfonate ion (⁻OMs), a methyl sulfate ion (⁻OSO₃Me), a chloride ion (Cl⁻), a bromide ion (Br⁻), an iodide ion (I⁻), a tetrafluoroborate ion (BF₄⁻), a hexafluorophosphate ion (PF₆⁻), a bis(trifluoromethanesulfonyl)imide ion ((CF₃SO₂)₂N⁻) and a trifluoroacetate ion (CF₃COO⁻), and further an anion selected from ⁻OTf, ⁻OSO₃Me, ⁻OTs, ⁻OMs, Cl⁻ and Br⁻.
<39> The compound according to any of <1> to <38>, wherein in the general formula (3), X³¹ is a chlorine atom, X³¹ being bonded to a carbon atom at position 2 or 6 of the pyridine ring, R³¹ is a 3,7-dimethyloctyl group, and A³¹ is ⁻OTf.
<40> The compound according to any of <1> to <38>, wherein in the general formula (3), X³¹ is a chlorine atom, X³¹ being bonded to a carbon atom at position 2 or 6 of the pyridine ring, R³¹ is a 3,7,11,15-tetramethylhexadecyl group, and A³¹ is ⁻OTf.

### Examples

### <NMR measurement conditions>

The conditions for NMR (¹H NMR) measurements performed in synthesis examples and examples are as follows.
· Device: VNMR 400MR DD2 (manufactured by Agilent Technologies, Inc.)
· Measurement temperature: 25°C
· Waiting time: 10 seconds
· Number of times of integration: 8 times
· Observation range: 6410.3 Hz
· Pulse: 45°
· Data point: 65536

### Synthesis example 1 <synthesis of butyl 2-chloronicotinate>

The subject compound (intermediate 1) was synthesized by the scheme below. The synthesis was performed using a 200-ml three neck eggplant flask.

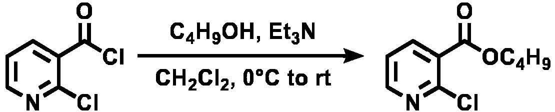

2.32 g (31.25 mmol, 1.1 eq.) of 1-butanol was added to a solution obtained from 5 g (28.41 mmol) of 2-chloronicotinic acid chloride and dichloromethane (56.82 ml), and this was cooled to 0°C. 4.31 g of triethylamine (42.62 mol, 1.5 eq.) was added dropwise thereto, and stirred at room temperature (25°C) for 1 hour. After the reaction ended, a saturated ammonium chloride aqueous solution was added to the reaction solution to stop the reaction, and extraction was carried out with dichloromethane. The obtained organic layer was washed with water and a saturated saline solution, and thereafter dried with anhydrous sodium sulfate and concentrated with a rotary evaporator to obtain a crude product. The obtained crude product was purified with silica gel column chromatography (hexane: ethyl acetate = 4:1 -> 1:1) to obtain the subject compound (intermediate 1) in an amount of 5.95 g (27.85 mmol, yield 98%). The ¹H NMR measurement results of the compound were as follows:
¹H NMR (400 MHz, CDCl₃) δ: 8.5 (1H, d, J = 4.8 Hz), 8.2 (1H, d, J = 8 Hz), 7.3 (1H, dd, J = 4.8, 8 Hz), 4.4 (2H, t, J = 6.4 Hz), 1.8-1.7 (2H, m), 1.5-1.4 (2H, m), 1.0 (3H, t, J = 7.6 Hz) ppm.

### Synthesis example 2 <synthesis of dodecyl 2-chloronicotinate>

The subject compound (intermediate 2) was synthesized by the scheme below. The synthesis was performed using a 200-mL three neck eggplant flask.

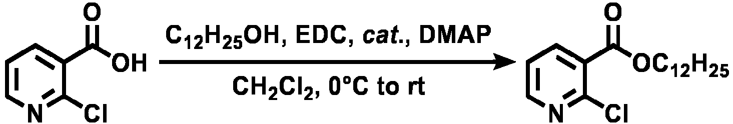

3.55 g (19.04 mmol, 1.0 eq.) of dodecanol was added to a solution obtained from 3 g (19.04 mmol) of 2-chloronicotinic acid and dichloromethane (38.08 ml), and this was cooled to 0°C. 0.12 g (0.95 mmol, 0.05 eq.) of 4-dimethylaminopyridine (DMAP) and further 4.38 g (22.85 mmol, 1.2 eq.) of 1-(3-dimethylaminopropyl)-3-ethylcarbodiimide hydrochloride (EDCl) were added thereto, and stirred at room temperature (25°C) all night. After the reaction ended, a saturated ammonium chloride aqueous solution was added to the reaction solution to stop the reaction, and extraction was carried out with dichloromethane. The obtained organic layer was washed with water and a saturated saline solution, and thereafter dried with anhydrous sodium sulfate and concentrated with a rotary evaporator to obtain a crude product. The obtained crude product was purified with silica gel column chromatography (hexane: ethyl acetate = 4:1 -> 1:1) to obtain the subject compound (intermediate 2) in an amount of 6.1 g (18.71 mmol, yield 98%). The ¹H NMR measurement results of the compound were as follows:
¹H NMR (400 MHz, CDCl₃) δ: 9.5 (1H, d, J = 6.4 Hz), 8.9 (1H, dd, J = 1.6, 8 Hz), 8.2 (1H, dd, J = 6, 7.6 Hz), 5.0 (2H, t, J = 7.6 Hz), 4.4 (2H, t, 6.8 Hz), 2.04-1.97 (2H, m), 1.87-1.77 (2H, m), 1.49-1.25 (11H, m), 0.90-0.86 (6H, m) ppm.

### Synthesis example 3 <synthesis of 2-decyltetradecyl 2-chloronicotinate>

The subject compound (intermediate 3) was synthesized by the scheme below. The synthesis was performed using a 200-mL three neck eggplant flask.

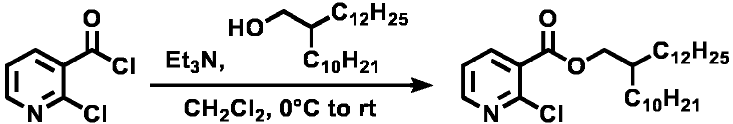

11.08 g (31.25 mmol, 1.1 eq.) of 2-decyl-1-tetradecanol was added to a solution obtained from 5 g (28.41 mmol) of 2-chloronicotinic acid chloride and dichloromethane (56.82 ml), and this was cooled to 0°C. 4.31 g of triethylamine (42.62 mol, 1.5 eq.) was added dropwise thereto, and stirred at room temperature (25°C) for 1 hour. After the reaction ended, a saturated ammonium chloride aqueous solution was added to the reaction solution to stop the reaction, and extraction was carried out with dichloromethane. The obtained organic layer was washed with water and a saturated saline solution, and thereafter dried with anhydrous sodium sulfate and concentrated with a rotary evaporator to obtain a crude product. The obtained crude product was purified with silica gel column chromatography (hexane: ethyl acetate = 4:1 -> 1:1) to obtain the subject compound (intermediate 3) in an amount of 13.8 g (27.85 mmol, yield 98%). The ¹H NMR measurement results of the compound were as follows:
¹H NMR (400 MHz, CDCl₃) δ: 8.51 (1H, dd, J = 2.4, 4.8 Hz), 8.12 (1H, dd, J = 2, 7.6 Hz), 7.33 (1H, dd, J = 4.4, 7.6 Hz), 4.28 (2H, d, 5.2 Hz), 1.79-1.76 (1H, m), 1.45-1.26 (40H, m), 0.88 (6H, t, J = 6.4 Hz) ppm.

### Synthesis example 4 <synthesis of N-butyl-2-chloronicotinamide>

The subject compound (intermediate 4) was synthesized by the scheme below. The synthesis was performed using a 100-mL three neck eggplant flask.

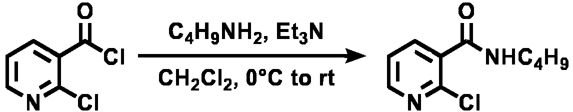

A dichloromethane (56.82 ml) solution of 2-chloronicotinic acid chloride (5 g, 28.41 mmol) was cooled to 0°C, and 2.18 g of butylamine (29.83 mmol, 1.05 eq.), and then, 4.31 g of triethylamine (42.62 mol, 1.5 eq.), were added dropwise thereto, and stirred at room temperature (25°C) for 1 hour. After the reaction ended, a saturated ammonium chloride aqueous solution was added to the reaction solution to stop the reaction, and extraction was carried out with dichloromethane. The obtained organic layer was washed with water and a saturated sodium chloride aqueous solution, and thereafter dried with anhydrous sodium sulfate. The organic layer was concentrated with a rotary evaporator, and this was purified with column chromatography (hexane: ethyl acetate = 10:1 -> 3:1) to obtain the subject compound (intermediate 4) in an amount of 5.74 g (2.70 mmol, yield 95%). The ¹H NMR measurement results of the compound were as follows:
¹H NMR (400 MHz, CDCl₃) δ: 8.5 (1H, d, J = 4.8 Hz), 8.1 (1H, d, J = 8 Hz), 7.4 (1H, dd, J = 4.8, 8 Hz), 6.5 (1H, s), 3.5(2H, q, J = 5.6, 7.2 Hz), 1.7-1.7 (2H, m), 1.6-1.4 (2H, m), 1.0 (3H, t, J = 7.6 Hz) ppm.

### Synthesis example 5 <synthesis of butyl trifluoromethanesulfonate)>

The subject compound (intermediate 5) was synthesized by the scheme below. The synthesis was performed using a 100-mL three neck eggplant flask.

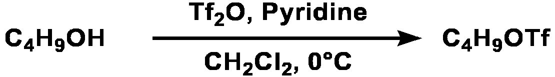

1 g (13.5 mmol) of butanol and dichloromethane (27 ml) were added to the eggplant flask, and this was cooled to 0°C. After that, trifluoromethanesulfonic anhydride (5.71 g, 20.25 mmol, 1.5 eq.) and pyridine (1.17 g, 14.9 mmol, 1.1 eq.) were added dropwise in succession, and reacted at 0°C for 1 hour under stirring. After the reaction ended, hexane was added to the reaction end solution, and the formed crystals were filtered and removed. After the obtained filtrate was concentrated with an evaporator, hexane was added to the residue, and the organic layer was washed with water and a saturated saline solution. After the organic layer was dried with anhydrous sodium sulfate, the organic layer was concentrated with an evaporator, and the obtained residue (containing the subject compound) was used for a reaction without being purified.

### Synthesis example 6 <synthesis of dodecyl trifluoromethanesulfonate)>

The subject compound (intermediate 6) was synthesized by the scheme below. The synthesis was performed using a 100-mL three neck eggplant flask.

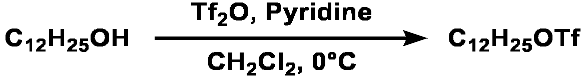

1 g (5.36 mmol) of dodecanol and dichloromethane (10.7 ml) were added to the eggplant flask, and this was cooled to 0°C. After that, trifluoromethanesulfonic anhydride (2.27 g, 8.04 mmol, 1.5 eq.) and pyridine (0.47 g, 5.9 mmol, 1.1 eq.) were added dropwise in succession, and reacted at 0°C for 1 hour under stirring. After the reaction ended, hexane was added to the reaction end solution, and the formed crystals were filtered and removed. After the obtained filtrate was concentrated with an evaporator, hexane was added to the residue, and the organic layer was washed with water and a saturated saline solution. After the organic layer was dried with anhydrous sodium sulfate, the organic layer was concentrated with an evaporator, and the obtained residue (containing the subject compound) was used for a reaction without being purified.

### Synthesis example 7 <synthesis of 3,7-dimethyloctyl trifluoromethanesulfonate>

The subject compound (intermediate 7) was synthesized by the scheme below. The synthesis was performed using a 100-mL three neck eggplant flask.

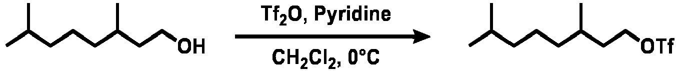

1 g (6.32 mmol) of 3,7-dimethyloctanol and dichloromethane (12.6 ml) were added to the eggplant flask, and this was cooled to 0°C. After that, trifluoromethanesulfonic anhydride (2.67 g, 9.48 mmol, 1.5 eq.) and pyridine (0.55 g, 6.95 mmol, 1.1 eq.) were added dropwise in succession, and reacted at 0°C for 1 hour under stirring. After the reaction ended, hexane was added to the reaction solution, and the formed crystals were filtered and removed. After the obtained filtrate was concentrated with an evaporator, hexane was added to the residue, and the organic layer was washed with water and a saturated saline solution. After the organic layer was dried with anhydrous sodium sulfate, the organic layer was concentrated with an evaporator, and the obtained residue (containing the subject compound) was used for a reaction without being purified.

### Synthesis example 8 <synthesis of 3,7,11,15-tetramethylhexadecyl trifluoromethanesulfonate>

The subject compound (intermediate 8) was synthesized by the scheme below. The synthesis was performed using a 50-mL three neck eggplant flask.

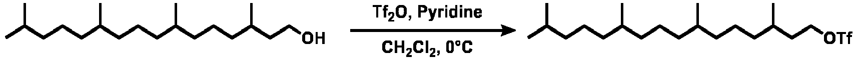

1 g (3.35 mmol) of 3,7,11,15-tetramethylhexadecanol and dichloromethane (6.7 ml) were added to the eggplant flask, and this was cooled to 0°C. After that, trifluoromethanesulfonic anhydride (1.42 g, 50.3 mmol, 1.5 eq.) and pyridine (0.29 g, 3.67 mmol, 1.1 eq.) were added dropwise in succession, and reacted at 0°C for 1 hour under stirring. After the reaction ended, hexane was added to the reaction end solution, and the formed crystals were filtered and removed. After the obtained filtrate was concentrated with an evaporator, hexane was added to the residue, and the organic layer was washed with water and a saturated saline solution. After the organic layer was dried with anhydrous sodium sulfate, the organic layer was concentrated with an evaporator, and the obtained residue (containing the subject compound) was used for a reaction without being purified.

### Synthesis example 9 <synthesis of tetracosyl trifluoromethanesulfonate)>

The subject compound (intermediate 9) was synthesized by the scheme below. The synthesis was performed using a 50-mL three neck eggplant flask.

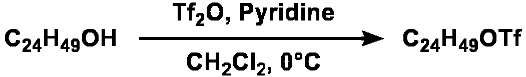

1 g (2.82 mmol) of tetracosanol and dichloromethane (5.64 ml) were added to the eggplant flask, and this was cooled to 0°C. After that, trifluoromethanesulfonic anhydride (1.19 g, 4.23 mmol, 1.5 eq.) and pyridine (0.25 g, 3.1 mmol, 1.1 eq.) were added dropwise in succession, and reacted at 0°C for 1 hour under stirring. After the reaction ended, hexane was added to the reaction end solution, and the formed crystals were filtered and removed. After the obtained filtrate was concentrated with an evaporator, hexane was added to the residue, and the organic layer was washed with water and a saturated saline solution. After the organic layer was dried with anhydrous sodium sulfate, the organic layer was concentrated with an evaporator, and the obtained residue (containing the subject compound) was used for a reaction without being purified.

### Example 1 <Synthesis of 3-(2-chloropyridin-1-ium-1-yl)propane-1-sulfonate>

The subject compound was synthesized by the scheme below. The synthesis was performed using a 100-mL one neck eggplant flask.

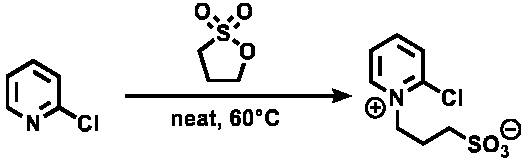

1,3-propanesultone (4.04 g, 33.03 mmol, 0.75 eq.) was added to 2-chloropyridine (5 g, 44.03 mmol), and stirred at 60°C for 5 hours. Acetone was added to the reaction end solution, the formed crystals were filtered off, and drying under reduced pressure was carried out to obtain the subject compound in an amount of 4.98 g (21.14 mmol, yield 64%). The ¹H NMR measurement results of the compound were as follows:
¹H NMR (400 MHz, D₂O) δ: 9.0 (1H, d, J = 6.4 Hz), 8.5 (1H, t, J = 6.4 Hz), 8.2 (1H, d, J = 8.4 Hz), 8.0 (1H, t, J = 8.4 Hz), 5.0 (2H, t, J = 8 Hz), 3.1 (2H, t, J = 7.2 Hz), 2.5 (2H, m) ppm.

### Example 2 <synthesis of 3-(butoxycarbonyl)-2-chloro-1-dodecylpyridin-1-ium trifluoromethanesulfonate>

The subject compound was synthesized by the scheme below. The synthesis was performed using a 100-mL one neck eggplant flask.

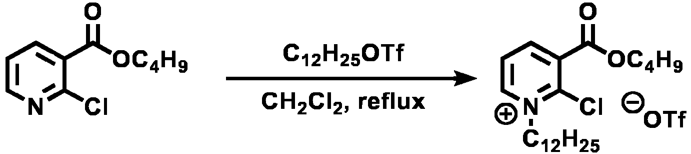

Dichloromethane (10.7 ml) and 1.03 g (4.82 mmol, 0.9 eq.) of intermediate 1 (butyl 2-chloronicotinate) synthesized in advance were added to intermediate 6 (dodecyl trifluoromethanesulfonate) obtained in a previous reaction, and stirred under reflux at 45°C (water bath temperature) for 24 hours. After the reaction ended, the reaction end solution was concentrated with an evaporator. Hexane was added to the obtained residue, and the formed crystals were collected. The obtained crystals were washed with diethyl ether to obtain the subject compound in an amount of 2.13 g (4.0 mmol, yield 83%). The ¹H NMR measurement results of the compound were as follows:
¹H NMR (400 MHz, CDCl₃) δ: 9.45 (1H, dd, J = 1.6, 6 Hz), 8.88 (1H, dd, J = 1.6, 8 Hz), 8.24 (1H, dd, J = 6, 8 Hz), 4.90 (2H, t, J = 8 Hz), 4.44 (2H, t, 6.8 Hz), 2.03-1.95 (2H, m), 1.82-1.75 (2H, m), 1.52-1.26 (20H, m), 0.98 (3H, t, J = 7.2 Hz), 0.88 (3H, t, J = 6.8 Hz) ppm.

### Example 3 <synthesis of 3-(dodecyloxycarbonyl)-2-chloro-1-dodecylpyridin-1-ium trifluoromethanesulfonate>

The subject compound was synthesized by the scheme below. The synthesis was performed using a 100-mL one neck eggplant flask.

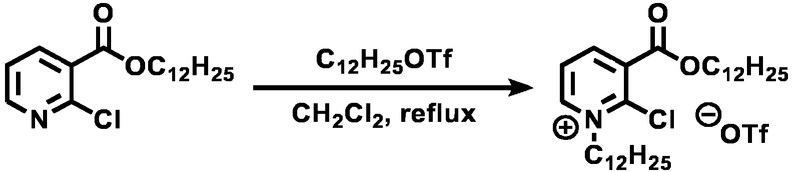

Dichloromethane (10.7 ml) and 1.57 g (4.82 mmol, 0.9 eq.) of intermediate 2 (dodecyl 2-chloronicotinate) synthesized in advance were added to intermediate 6 (dodecyl trifluoromethanesulfonate) obtained in a previous reaction, and stirred under reflux at 45°C (water bath temperature) for 24 hours. After the reaction ended, the reaction end solution was concentrated with an evaporator. Hexane was added to the obtained residue, and the formed crystals were collected. The obtained crystals were washed with diethyl ether to obtain the subject compound in an amount of 1.93 g (3.0 mmol, yield 62%). The ¹H NMR measurement results of the compound were as follows:
¹H NMR (400 MHz, CDCl₃) δ: 9.59 (1H, dd, J = 1.6, 6.4 Hz), 8.83 (1H, dd, J = 1.6, 8 Hz), 8.20 (1H, dd, J = 6, 8 Hz), 4.97 (2H, t, J = 8 Hz), 4.45 (2H, t, 6.8 Hz), 2.05-1.97 (2H, m), 1.84-1.77 (2H, m), 1.47-1.21 (36H, m), 0.88 (6H, t, J = 6.8 Hz) ppm.

### Example 4 <synthesis of 3-(2-decyltetradecyloxycarbonyl)-2-chloro-1-dodecylpyridin-1-ium trifluoromethanesulfonate>

The subject compound was synthesized by the scheme below. The synthesis was performed using a 100-mL one neck eggplant flask.

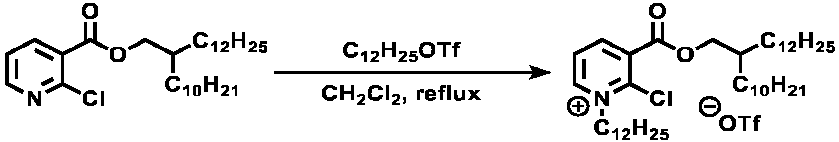

Dichloromethane (10.7 ml) and 2.4 g (4.82 mmol, 0.9 eq.) of intermediate 3 (2-decyltetradecyl 2-chloronicotinate) synthesized in advance were added to intermediate 6 (dodecyl trifluoromethanesulfonate) obtained in a previous reaction, and stirred under reflux at 45°C (water bath temperature) for 24 hours. After the reaction ended, the reaction end solution was concentrated with an evaporator. Hexane was added to the obtained residue, and the formed crystals were collected. The obtained crystals were washed with diethyl ether to obtain the subject compound in an amount of 2.0 g (2.46 mmol, yield 51%). The ¹H NMR measurement results of the compound were as follows:
¹H NMR (400 MHz, CDCl₃) δ: 9.56 (1H, dd, J = 1.6, 6.4 Hz), 8.83 (1H, dd, J = 1.6, 8 Hz), 8.25 (1H, dd, J = 6.4, 8.4 Hz), 4.95 (2H, t, J = 7.6 Hz), 4.36 (2H, d, J = 5.6 Hz), 2.02-1.97 (2H, m), 1.81-1.78 (1H, m), 1.48-1.22 (61H, m), 0.90-0.86 (6H, m) ppm.

### Example 5 <synthesis of 1-butyl-2-chloro-3-((dodecyloxy)carbonyl)pyridin-1-ium trifluoromethanesulfonate>

The subject compound was synthesized by the scheme below. The synthesis was performed using a 200-mL one neck eggplant flask.

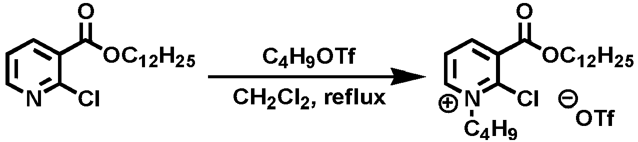

Dichloromethane (26.97 ml) and 3.95 g (12.14 mmol, 0.9 eq.) of intermediate 2 (dodecyl 2-chloronicotinate) synthesized in advance were added to intermediate 5 (butyl trifluoromethanesulfonate) obtained in a previous reaction, and stirred under reflux at 45°C (water bath temperature) for 24 hours. After the reaction ended, the reaction end solution was concentrated with an evaporator. Hexane was added to the obtained residue, and the formed crystals were collected. The obtained crystals were washed with diethyl ether to obtain the subject compound in an amount of 3.25 g (6.1 mmol, yield 50%). The ¹H NMR measurement results of the compound were as follows:
¹H NMR (400 MHz, CDCl₃) δ: 9.50 (1H, dd, J = 1.6, 6 Hz), 8.82 (1H, dd, J = 1.6, 8 Hz), 8.24 (1H, dd, J = 6, 7.6 Hz), 4.94 (2H, t, J = 8 Hz), 4.43 (2H, t, 6.8 Hz), 2.03-1.95 (2H, m), 1.83-1.76 (2H, m), 1.53-1.21 (20H, m), 1.01 (3H, t, J = 7.2 Hz), 0.88 (3H, t, J = 6.4 Hz) ppm.

### Example 6 <synthesis of 1-tetracosyl-2-chloro-3-((dodecyloxy)carbonyl)pyridin-1-ium trifluoromethanesulfonate>

The subject compound was synthesized by the scheme below. The synthesis was performed using a 200-mL one neck eggplant flask.

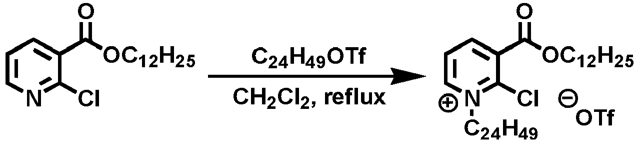

Dichloromethane (26.97 ml) and 3.95 g (12.14 mmol, 0.9 eq.) of intermediate 2 (dodecyl 2-chloronicotinate) synthesized in advance were added to intermediate 9 (tetracosyl trifluoromethanesulfonate) obtained in a previous reaction, and stirred under reflux at 45°C (water bath temperature) for 24 hours. After the reaction ended, the reaction end solution was concentrated with an evaporator. Hexane was added to the obtained residue, and the formed crystals were collected. The obtained crystals were washed with diethyl ether to obtain the subject compound in an amount of 7.3 g (9.0 mmol, yield 74%). The ¹H NMR measurement results of the compound were as follows:
¹H NMR (400 MHz, CDCl₃) δ: 9.55 (1H, d, J = 6.4 Hz), 8.85 (1H, dd, J = 1.6, 8 Hz), 8.22 (1H, dd, J = 6, 7.6 Hz), 4.95 (2H, t, J = 7.6 Hz), 4.44 (2H, t, 6.8 Hz), 2.04-1.97 (2H, m), 1.87-1.77 (2H, m), 1.50-1.21 (60H, m), 0.88 (6H, t, J = 6.4 Hz) ppm.

### Example 7 <synthesis of 1-butyl-3-(butylcarbamoyl)-2-chloropyridin-1-ium trifluoromethanesulfonate>

The subject compound was synthesized by the scheme below. The synthesis was performed using a 100-mL one neck eggplant flask.

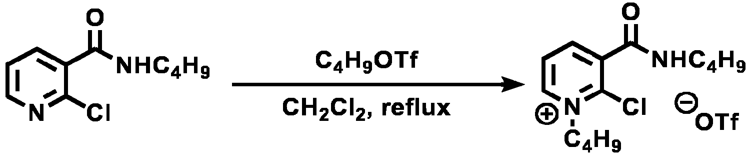

Dichloromethane (10.7 ml) and 1.03 g (4.82 mmol, 0.9 eq.) of intermediate 4 (N-butyl-2-chloronicotinamide) synthesized in advance were added to intermediate 5 (butyl trifluoromethanesulfonate) obtained in a previous reaction, and stirred under reflux at 45°C (water bath temperature) for 24 hours. After the reaction ended, the reaction end solution was concentrated with an evaporator. Hexane was added to the obtained residue, and the formed crystals were collected. The obtained crystals were washed with diethyl ether to obtain the subject compound in an amount of 0.79 g (1.88 mmol, yield 39%). The ¹H NMR measurement results of the compound were as follows:
¹H NMR (400 MHz, CDCl₃) δ: 9.00 (1H, dd, J = 1.2, 6.4 Hz), 8.38 (1H, dd, J = 1.2, 7.6 Hz), 8.02 (1H, t, J = 5.6 Hz), 7.96 (1H, td, J = 6.4, 7.6 Hz), 4.72 (2H, t, J = 7.6 Hz), 3.42 (2H, dd, J = 6.8, 12.8 Hz), 1.99-1.91 (2H, m), 1.64-1.57 (2H, m), 1.51-1.37 (4H, m), 1.01-0.93 (6H, m) ppm.

### Example 8 <synthesis of 3-(butylcarbamoyl)-2-chloro-1-dodecylpyridin-1-ium trifluoromethanesulfonate>

The subject compound was synthesized by the scheme below. The synthesis was performed using a 100-mL one neck eggplant flask.

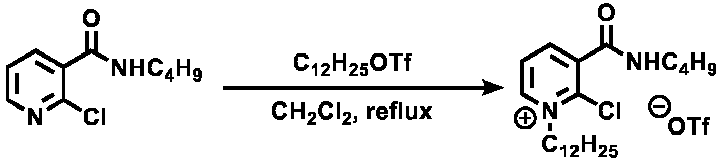

Dichloromethane (10.7 ml) and 1.03 g (4.82 mmol, 0.9 eq.) of intermediate 4 (N-butyl-2-chloronicotinamide) synthesized in advance were added to intermediate 6 (dodecyl trifluoromethanesulfonate) obtained in a previous reaction, and stirred under reflux at 45°C (water bath temperature) for 24 hours. After the reaction ended, the reaction end solution was concentrated with an evaporator. Hexane was added to the obtained residue, and the formed crystals were collected. The obtained crystals were washed with diethyl ether to obtain the subject compound in an amount of 0.91 g (1.71 mmol, yield 35%). The ¹H NMR measurement results of the compound were as follows:
¹H NMR (400 MHz, CDCl₃) δ: 8.99 (1H, dd, J = 1.2, 6 Hz), 8.37 (1H, dd, J = 1.2, 7.6 Hz), 8.04 (1H, t, J = 5.6 Hz), 7.95 (1H, dd, J = 6.4, 8 Hz), 4.71 (2H, t, J = 8 Hz), 3.42 (2H, dd, J = 6.8, 12.8 Hz), 2.03-1.92 (2H, m), 1.65-1.57 (2H, m), 1.46-1.24 (20H, m), 0.97-0.88 (6H, m) ppm.

### Example 9 <synthesis of 2-chloro-1-(3,7-dimethyloctyl)pyridin-1-ium trifluoromethanesulfonate>

The subject compound was synthesized by the scheme below. The synthesis was performed using a 100-mL one neck eggplant flask.

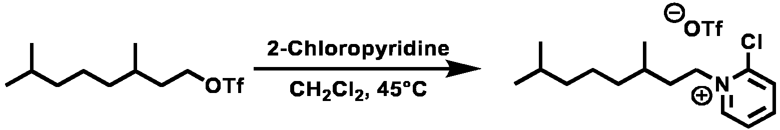

Dichloromethane (7.62 ml) and 2-chloropyridine (0.6 g, 3.81 mmol) were added to 1.22 g (4.2 mmol, 1.1 eq.) of intermediate 7 (3,7-dimethyloctyl trifluoromethanesulfonate) obtained in a previous reaction, and stirred at 45°C for 24 hours. After the reaction ended, the reaction solution was concentrated with an evaporator to obtain the subject compound in an amount of 1.5 g (3.71 mmol, yield 97%). The ¹H NMR measurement results of the compound were as follows:
¹H NMR (400 MHz, CDCl₃) δ: 9.1 (1H, d, J = 6 Hz), 8.5 (1H, t, J = 6.4 Hz), 8.1 (2H, m), 4.8 (2H, m), 1.9 (1H, m), 1.8 (1H, m), 1.6 (1H, m), 1.5 (1H, m), 1.4-1.2 (6H, m), 1.0 (3H, t, J = 6.8 Hz), 0.9 (6H, m) ppm.

### Example 10 <synthesis of 2-chloro-1-(3,7,11,15-tetramethylhexadecyl)pyridin-1-ium trifluoromethanesulfonate>

The subject compound was synthesized by the scheme below. The synthesis was performed using a 50-mL one neck eggplant flask.

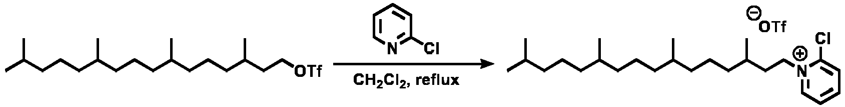

Dichloromethane (6.7 ml) and 2-chloropyridine (0.35 g, 3.05 mmol) were added to 1.44 g (1.1 eq.) of intermediate 8 (3,7,11,15-tetramethylhexadecyl trifluoromethanesulfonate) obtained in a previous reaction, and stirred under reflux at 45°C (water bath temperature) for 24 hours. After the reaction ended, the reaction end solution was concentrated with an evaporator. The obtained residue was purified with preparative TLC to obtain the subject compound in an amount of 0.38 g (0.7 mmol, yield 23%). The ¹H NMR measurement results of the compound were as follows:
¹H NMR (400 MHz, CDCl₃) δ: 9.24 (1H, dd, J = 1.6, 6 Hz), 8.52 (1H, dt, J = 1.6, 8.4 Hz), 8.1 (1H, t, J = 6, Hz), 8.04 (1H, dd, J = 1.2, 8 Hz), 4.82 (2H, m), 2.00-1.92 (1H, m), 1.84-1.74 (1H, m), 1.69-1.64 (1H, m), 1.57-1.47 (1H, m), 1.40-1.02 (20H, m), 0.90-0.84 (15H, m) ppm.

### <Examination example 1>

### (1) Reagent

The reagents used in examination example 1 were as follows:
· phosphoric acid (85%): manufactured by FUJIFILM Wako Pure Chemical Corporation
· disodium hydrogen phosphate: manufactured by FUJIFILM Wako Pure Chemical Corporation
· potassium dihydrogen phosphate: manufactured by FUJIFILM Wako Pure Chemical Corporation
· ethanol (99.5%): manufactured by FUJIFILM Wako Pure Chemical Corporation
· curcumin: manufactured by FUJIFILM Wako Pure Chemical Corporation
· acetonitrile: manufactured by FUJIFILM Wako Pure Chemical Corporation
· hydrogen peroxide (30%): manufactured by FUJIFILM Wako Pure Chemical Corporation

Those shown in parenthesis are effective amounts. The amounts described below were expressed in terms of effective amounts.

### (2) Evaluation of oxidative decomposition activity

Oxidative decomposition activity was evaluated using 3-(2-chloropyridin-1-ium-1-yl)propane-1-sulfonate of example 1 (hereinafter referred to as the pyridinium compound of example 1).

A solution of 5.29 mM of the pyridinium compound of example 1 (which is referred to as solution A) was prepared using ion-exchanged water, a solution of 1765 mM of hydrogen peroxide (which is referred to as solution B) was prepared using ion-exchanged water and 30% hydrogen peroxide, and a solution of 1.32 mM of curcumin was prepared using ethanol. 0.1 ml of solution B and 1 ml of solution A were added to 18.8 ml of a phosphate buffer solution adjusted to pH = 7.3 and stirred for 10 seconds, and then, 0.1 ml of the curcumin solution was added thereto and stirred for 10 minutes (the final concentrations of the pyridinium compound of example 1, hydrogen peroxide and curcumin were 0.264 mM, 8.824 mM and 0.0066 mM, respectively). The phosphate buffer solution with pH = 7.3 was prepared from disodium hydrogen phosphate and potassium dihydrogen phosphate. 10 minutes later, 0.5 ml of the reaction solution was taken, and 0.5 ml of a quenching liquid (0.5 M aqueous phosphate solution/acetonitrile = 1:1 (vol)) was added thereto to obtain a sample solution. This was quantified with HPLC to determine a remaining amount of curcumin.

As a result of the quantification with HPLC, oxidative decomposition activity of the pyridinium compound of example 1 under the neutral condition was 100%. The HPLC conditions were as shown below. Note that, when the compounds of examples 2 to 10 are used in place of the pyridinium compound of example 1 and evaluated in the same manner, they are equal in obtained oxidative decomposition activity to the pyridinium compound of example 1.

### <HPLC conditions>

· Device
   SHIMADZU CBM-20A
   pump: LC-20A, UV-Vis detector: SPD-20A, PDA detector: SPD-M20A, column oven: CTO-20A, auto sampler: SIL-20A
· Analysis conditions
   column: L-column ODS, 150 mm × 4.6 mm, 5 µm (Chemicals Evaluation and Research Institute, Japan), oven temperature: 40°C, detector: UV (430 nm), injection volume: 10 µl, flow rate: 1.0 mL/min, mobile phase: A = 50 mM phosphoric acid, B = acetonitrile

## Claims

1. A novel pyridinium compound represented by the following general formula (1), (2) or (3): 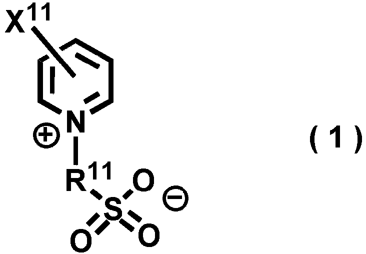
wherein X¹¹ is a halogen atom, X¹¹ being bonded to a carbon atom at position 2, 4 or 6 of the pyridine ring, and R¹¹ is an alkylene group with 1 or more and 24 or less carbons which may contain a heteroatom; 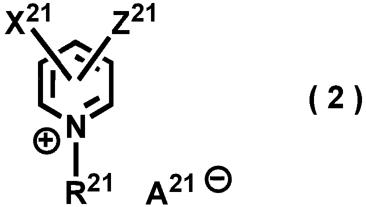
wherein X²¹ is a halogen atom, X²¹ being bonded to a carbon atom at position 2, 4 or 6 of the pyridine ring, Z²¹ is a group selected from -COO-R²², -CONH-R²³ and - CON(R²⁴)(R²⁵), Z²¹ being bonded to a carbon atom of the pyridine ring different from the carbon atom to which X²¹ is bonded, R²¹ is an alkyl group with 3 or more and 24 or less carbons which may contain a heteroatom, R²², R²³, R²⁴ and R²⁵ each represent an alkyl group with 3 or more carbons which may contain a heteroatom, and A²¹⁻ is an anion; or 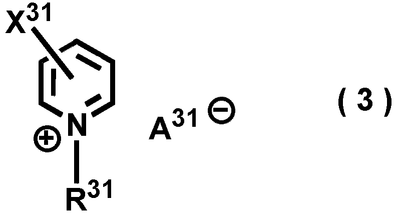
wherein X³¹ is a halogen atom, X³¹ being bonded to a carbon atom at position 2, 4 or 6 of the pyridine ring, R³¹ is a branched alkyl group with 10 or more and 24 or less carbons which may contain a heteroatom, and A³¹⁻ is an anion.

2. The compound according to claim 1, wherein in the general formula (1), -R¹¹-SO₃ is -CH₂-(CH₂)ₙ₁₁-Y¹-(CH₂)ₙ₁₂-SO₃, Y¹ is a single bond or a heteroatom, n11 and n12 each represent an integer of 0 or more and 23 or less, and a total of n11 and n12 is 0 or more and 23 or less.

3. The compound according to claim 1 or 2, wherein in the general formula (2), -R²¹ is -CH₂-(CH₂)ₙ₂₁-Y²- (CH₂)ₙ₂₂-CH₃, Y² is a single bond or a heteroatom, n21 and n22 each represent an integer of 0 or more and 22 or less, and a total of n21 and n22 is 1 or more and 22 or less.

4. The compound according to any of claims 1 to 3, wherein in the general formula (3), -R³¹ is -CH₂-R³¹¹-Y³-R³¹²-CH₃, Y³ is a single bond or a heteroatom, R³¹¹ and R³¹² each represent a single bond or an alkylene group, a total number of carbons in R³¹¹ and R³¹² is 8 or more and 22 or less, and at least one of R³¹¹ and R³¹² is a branched alkylene group.

5. The compound according to any of claims 1 to 4, wherein in the general formula (1), R¹¹ is an alkylene group with 1 or more and 24 or less carbons not containing a heteroatom.

6. The compound according to any of claims 1 to 5, wherein in the general formula (2), R²¹ is an alkyl group with 3 or more and 24 or less carbons not containing a heteroatom.

7. The compound according to any of claims 1 to 6, wherein in the general formula (2), Z²¹ is a group selected from -COO-R²² and -CONH-R²³.

8. The compound according to any of claims 1 to 7, wherein in the general formula (3), R³¹ is a branched alkyl group with 10 or more and 24 or less carbons not containing a heteroatom.
